# EUROPEAN PATENT APPLICATION

(11) **EP 4 538 373 A1**
(43) Date of publication of application: **16.04.2025**
(21) Application number: 23203413.2
(22) Date of filing: 13.10.2023
(51) Int. Cl.: C12N 15/10, C07C 39/06

(54) **ISOLATION OF NUCLEIC ACIDS FROM BIOLOGICAL SAMPLES**

(71) Applicant: AXAGARIUS GmbH & Co. KG, 52355 Düren (DE)
(72) Inventor: Wyrich, Ralf, 41516 Grevenbroich (DE)
(74) Representative: dompatent von Kreisler Selting Werner - Partnerschaft von Patent- und Rechtsanwälten mbB

(57) **Abstract**

The current invention relates to the isolation of nucleic acids from sample materials present or preserved in chaotropic salt containing solutions and suitable kits therefore.

## Description

The current invention relates to the isolation of nucleic acids from sample materials present or preserved in chaotropic salt containing solutions and a suitable kit therefore.

### Background of the Invention

The preservation of biological sample materials gets increasing attention in the field of molecular biology and molecular diagnostics. The finding that biological samples change during storage and/or shipment to the analyzing laboratory over time generated the need to develop stabilization solutions for different sample materials and molecular analytes. One example for a specific system is the PAXgene Blood RNA tube (PreAnalytiX GmbH, Hombrechtikon, Switzerland) which stabilizes specifically the intracellular RNA in white blood cells in human whole blood. Another example is the DNA/RNA Shield^{™} stabilization solution from Zymo Research (Irvine, USA) containing the chaotropic salt guanidinium thiocyanate. This stabilization solution preserves DNA and RNA in different sample materials like blood and saliva, but also stool samples.

It is a known fact, that stabilized biological samples can change their constitution, i.e., "alter", under the impact of the stabilization solution and its ingredients, over time and as a consequence, it can be problematic to extract nucleic acids from the stabilized, altered sample material. This is the case for the chaotropic salt-containing DNA/RNA Shield^{™} stabilization solution referred to above and also for cell-lysis products obtained by lysis with chaotropic salts that are not subjected to direct isolation of the nucleic acids after lysis. Moreover, the stabilization solution itself may interfere with the extraction kit used. The kits for extraction of nucleic acids from biological samples were normally developed with fresh samples in the past. It can therefore be problematic, if a new stabilization technology comes up, that changes the constitution of the sample material in a way that the extraction technology cannot cope with.

An example for a DNA extraction kit that was developed with fresh sample material and could not cope with stabilized sample materials was the NucleoSpin 96 DNA Stool kit (MACHEREY-NAGEL GmbH & Co.KG, Dueren, Germany) used in conjunction with stool samples stored in Zymo DNA/RNA Shield stabilization solution. The NucleoSpin 96 DNA Stool kit uses silica membrane technology in a 96 well format to isolate DNA from stool samples.

When human stool samples, preserved in the Zymo DNA/RNA Shield stabilization solution, were processed with the standard protocol of the NucleoSpin 96 DNA Stool kit, the samples gave almost no yield of DNA. Even the addition of very little amounts of DNA/RNA Shield solution to the stool sample reduced the DNA-yield already as can be seen in Reference Example 1. It seemed, that the reagent interfered with the binding chemistry of the kit so that the DNA was not extracted efficiently.

A common method for the extraction of nucleic acids from crude samples is the use of a mixture of phenol and guanidinium salt (a chaotropic salt), which is commercially available from different vendors e.g. as TRIzol^{™} Reagent (ThermoFisher Scientific), QIAzol^{™} Lysis Reagent (QIAGEN) or NucleoZOL (MACHEREY-NAGEL GmbH & Co.KG, Dueren, Germany). The mixture of phenol and guanidinium salt is capable to disrupt hard to lyse samples like very protein or fat-rich samples. NucleoZOL reagent can also be used to neutralize the DNA/RNA Shield reagent via a simple pretreatment (see Reference Example 2) and recover the yield of DNA from stabilized stool samples.

With the pretreatment with NucleoZOL the average yield of DNA from stool samples can be restored, even in the presence of a large volume of DNA/RNA Shield reagent. However, the downside is that NucleoZOL is a highly toxic phenol-containing solution. Guanidinium salts are hazardous, but phenol is mutagenic, carcinogenic, corrosively and needs to be handled under a fume hood. It was therefore desirable to find a reagent less toxic than NocleoZOL, to neutralize the negative effects of the DNA/RNA Shield reagent.

There was a need for a less- or non-toxic solution that is capable to neutralize negative impacts of the DNA/RNA Shield solution on the extraction of genomic DNA from stool samples using a silica membrane-based extraction kit like the NucleoSpin 96 DNA Stool kit. The procedure should work as with NucleoZOL reagent as a simple pretreatment of the stabilized stool sample after which the regular protocol can be carried out without any significant changes.

### Short Description of the Invention

It was now found that the negative impact of the stabilizing solution can be neutralized by the use of certain alkylphenol compounds, notably phenol compounds having at least one middle chain alkyl group such as eugenol, thymol and carvacrol. The use of eugenol in biological or pharmaceutical applications is known in EP 1463810B1; WO 002012069073A1; WO 002003054177A2; DE112015003039T5. Thus, as a first aspect of the invention, provided is a method for the isolation of nucleic acids from chaotropic salt-containing biological samples, the method comprising adding to the crude chaotropic salt-containing biological sample, prior to separation of the nucleic acids from said solution, a masking solution comprising a C₃₋₁₀ alkyl or C₃₋₁₀ alkenyl phenol component in an organic solvent to mask chaotropic salt(s) present in the crude sample

As a second aspect of the invention provided is a kit for isolating nucleic acids from a biological sample, comprising a masking solution comprising a C₃₋₁₀ alkyl or C₃₋₁₀ alkenyl phenol component in an organic solvent to mask chaotropic salt(s) present in the crude biological sample.

As a third aspect of the invention, provided is the use of a masking solution comprising a C₃₋₁₀ alkyl or C₃₋₁₀ alkenyl phenol component in an organic solvent to mask chaotropic salt(s) present in crude biological samples in a nucleic acid isolation method.

### Short Description of the Figures

Figure 1: DNA integrity testing via agarose gel electrophoresis of Example 1.
Figure 2: Sample analysis via end-point PCR of Example 1.
Figure 3: DNA integrity testing via agarose gel electrophoresis of Example 2.

### Detailed Description of the Invention

There are different chemical compounds that are based on the chemical basic structure of phenol and can be used in the method of the first aspect of the invention, but due to chemical modifications at the benzene ring, they are less toxic than phenol itself. In particular, the C₃₋₁₀ alkyl- or C₃₋₁₀ alkenylphenol component has the formula (I) 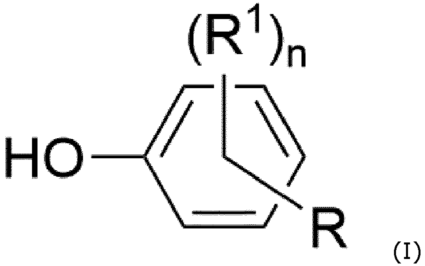 wherein
R is a straight- or branched-chain C₃₋₁₀ alkyl residue or C₃₋₁₀ alkenyl residue,
R¹ is independently selected from each other from a methyl, ethyl and methoxy group, and
n is an integer from 0 to 3,
preferably R is a straight- or branched-chain C₃₋₆ alkyl residue or C₃₋₆ alkenyl residue, R¹ is a methyl or methoxy group and n is 0 or 1.

The straight or branched chain C₃₋₁₀ alkyl residue include, but are not limited to, n-propyl, isopropyl, n-butyl, sec-butyl, tert-butyl, n-pentyl, sec-pentyl, neo-pentyl, n-hexyl and the like residues. The straight or branched chain C₃₋₁₀ alkenyl residue includes the above-mentioned specific alkyl residues having one to three double bonds

The compound of formula (I) may be naturally occurring or of synthetically produced. For instance, plants produce phytochemicals based on the basic phenol structure.

Eugenol (4-prop-2-enyl-2-methoxyphenol; CAS 97-53-0) is a phytochemical based on a phenol structure. It is produced by cloves (Eugenia aromatic) and other plants. It can be extracted from oil, generated from these plants. Eugenol is generally used in the cosmetic industry as a perfume component and in dentistry as analgesic, antibacterial and anti-inflammatory drug. Eugenol is in high concentrations irritant to eye and skin but is neither corrosive, nor carcinogenic. It is therefore much less critical in handling compared to phenol and does not need to be handled under a fume hood. Eugenol is poorly soluble in water and therefore ethanol is the preferred solvent to dilute eugenol in solution.

Eugenol was examined as a substitute for NucleoZOL to neutralize negative effects of the DNA/RNA Shield reagent on the extraction of DNA from stool samples. In order to increase the solubility of eugenol, it was used as a 1:1 (v/v) mixture with ethanol. As for the use of NucleoZol, eugenol was used as a solution for a pretreatment of a stool sample, that was dissolved in DNA/RNA Shield solution and subsequently, a standard kit was used to extract the DNA from the pretreated sample.

Thymol (2-isopropyl-5-methylphenol; CAS 89-83-8), is a second example for a phytochemical based on a phenol structure. It is produced by thyme (*Thymus vulgaris*) and other plants. It is generally used in skin disinfectants or mouthwashes, as it is bactericidal and fungicidal. As it is also poorly soluble in water, it was used as a 50% (w/v) solution dissolved in ethanol.

Another example is carvacrol (5-isopropyl-2-methylphenol, isothymol, CAS 499-75-2), which is produced by thyme (*Thymus vulgaris*) and other plants.

In a preferred embodiment of the method of the invention, the organic solvent is an alcohol, preferably is ethanol or propanol.

In another preferred embodiment of the method of the invention, the C₃₋₁₀ alkyl or C₃₋₁₀ alkenylphenol component is comprised in the organic solvent in at 10 to 90 % (w/v) or (v/v), preferably at 30 to 70 % (w/v) or (v/v), most preferably at about 50 % (w/v) or (v/v). Whether the above percentages in (w/v) or in (v/v) are applicable depends on the physical state of the phenol component. For liquid phenol components such as eugenol v/v is applicable, whereas for solid phenol components such as thymol w/v is applicable.

In another preferred embodiment of the method of the invention the volume ratio of the crude sample to the masking solution is from 10:1 to 1:10, preferably from 4:1 to 1:4, most preferably at about 2.5:1.

In still another preferred embodiment of the method of the invention, the weight ratio of chaotropic salt to the C₃₋₁₀ alkyl- or C₃₋₁₀ alkenylphenol component is from 3:1 to 1:3, preferably 2:1 to 1:2, most preferably is about 1:1. In the method of the invention, the crude chaotropic salt-containing biological sample may be a stool sample, cell lysate, environmental- or blood sample. The nucleic acids isolated by the method of the invention may be a DNA or RNA.

In a particular embodiment, the method of the invention comprises
(a) providing the crude chaotropic salt-containing biological sample;
(b) adding an appropriate amount of a masking solution as defined hereinbefore;
(c) optionally mechanically disrupting and clearing the masking solution containing sample of step (b);
(d) adding a binding solution to the solution obtained in step (b) or (c), bringing the resulting solution into contact with a nucleic acid-binding solid phase and separating the solid phase with the nucleic acids bound thereto from the remainder of the solution;
(e) washing the separated solid phase with the nucleic acids bound thereto at least once with same or different washing solutions; and
(f) desorbing the nucleic acids from the washed solid phase by adding an elution buffer.

In a specific embodiment, the method is for the separation of nucleic acids from stool samples and comprises a disrupting and clearing step (c), preferably the clearing step (c) comprises adding a dilution solution, mixing/incubating the resulting solution, centrifuging the resulting solution and subjecting an aliquot of the resulting supernatant to a filter device, most preferably the dilution solution comprises a non-chaotropic salt. Suitable diluting solutions for the clearing step (c) comprise sodium or potassium acetate in the range of 45-60% (w/v).

In another specific embodiment, the method is for the separation of nucleic acids from cell lysates lysed and/or stabilized by means of chaotropic salts, notably cell lysates that were kept on the chaotropic salts for a certain period of time, sufficient for causing the chaotropic salts to change the constitution of the nucleic acid material in the cell lysate. Depending on the circumstances, the change of the constitution may even start after 15 to 30 min.

In the above-mentioned specific embodiments, the binding solution may be an aqueous high salt solution, optionally also containing an alcohol and/or ionic or non-ionic detergent. A particularly preferred binding solution contains a chaotropic salt and a citrate buffer, preferably about 45-60 % (w/v) guanidiniumthiocyanat and about 5-30 % (w/v) sodium citrate. Alternatively, chaotropic salt-free binding solutions as known in the art may also be used.

The nucleic acid-binding solid phase may be a silica-matrix/membrane or magnetic particles.

Suitable matrices/membranes include silica membranes, suitable magnetic particles include superparamagnetic beads, preferably carboxylated magnetic particles.

In the method of the invention, the washing solutions of step (e) may be selected from washing solutions containing an alcohol, a chaotropic salt or mixtures thereof. In the method of the invention, the elution buffer of step (f) may be a slightly alkaline low-salt buffer, preferably the volume of the elution buffer may be about 10 to about 20 times the volume of the nucleic acid-binding solid phase employed in step (d).

The kit of the second aspect of the invention is particularly suitable for performing a method as defined hereinbefore.

In a preferred embodiment, the kit is for the separation of nucleic acids from stool samples and optionally further contains one or more of a dilution solution, a stool filter, a binding solution, a nucleic acid-binding solid phase, washing solutions and an elution buffer as defined hereinbefore.

In another preferred embodiment, the kit is for the separation of nucleic acids from cell lysates and optionally further contains one or more of a chaotropic salt-containing lysis solution, chaotropic salt-containing stabilizing solution, a binding solution, a nucleic acid-binding solid phase, washing solutions and an elution buffer as defined hereinbefore.

The invention is further described in the following Examples and Reference Examples, which are however not to be construed as limiting the invention.

### Examples

### Materials and Methods

The following materials were used in the examples below:
DNA/RNA Shield^{™} solution (Cat.No. R1100; Zymo Research) containing about 1.5 to 3 M guanidinium thiocyanate;
Bead Tubes Type A (MACHEREY-NAGEL GmbH & Co.KG, Dueren, Germany, Cat.No. 740786);
NucleoSpin 96 DNA Stool kit (Cat. No. 740473, MACHEREY-NAGEL GmbH & Co.KG, Dueren, Germany, buffer ST1 containing 1-5 % (w/v) of an ionic detergent; buffer ST2 containing sodium or potassium acetate in the range of 45-60 % (w/v); Buffer ST3 containing 45-60 % (w/v) guanidiniumthiocyanat; buffer ST4 containing 35-50 % (w/v) guanidiniumhydrochlorid and 20-35 % (v/v) isopropanol; buffer ST5 containing 80% (v/v) ethanol; the elution buffer containing a 5 mM Tris/HCl buffer, pH 8.5; NucleoSpin Stool Filter Plate containing a fibrous filter membrane, e.g. synthetic needled felt or silica fiber; and NucleoSpin Stool Binding Plate containing a silica fiber-separation membrane;
NucleoVac 96 vacuum manifold (Cat. No. 740681, MACHEREY-NAGEL GmbH & Co.KG, Dueren, Germany);
NucleoZOL (Cat. No. 740404, MACHEREY-NAGEL GmbH & Co.KG, Dueren, Germany) containing phenol and a chaotropic salt;
Retsch MM300 bead mill (Retsch, Haan, Germany);
Vortex Genie 2 (Scientific Industries);
Eugenol (Aldrich Cat. No. E51791);
Thymol (Sigma Cat. No. T0501);
Biotaq^{™} DNA polymerase and reaction buffer (Bioline Cat. No. BIO-21040), the buffer containing ammonium and magnesium chloride;
HDGreen Plus DNA/RNA stain (Intas Science Imaging); and
GeneRuler^{™}1kb Ladder (Thermo).

### Reference Example 1

### A. Spiking of DNA/RNA Shield^{™} stabilization solution (Zymo Research) into human stool samples

Sample material: Approx. 200 mg human stool was aliquoted into Bead Tubes Type A (MACHEREY-NAGEL).
Sample treatment: Increasing amounts of DNA/RNA Shield solution were added to the stool samples.

| | |
|---|---|
| Samples 1-4 | no DNA/RNA Shield solution |
| Samples 5-8 | addition of 10 µl DNA/RNA Shield solution |
| Samples 9-12 | addition of 20 µl DNA/RNA Shield solution |
| Samples 13-16 | addition of 30 µl DNA/RNA Shield solution |
| Samples 17-20 | addition of 40 µl DNA/RNA Shield solution |

### B. DNA-Extraktion using the the NucleoSoin 96 DNA Stool kit:

Add 950 µl Buffer ST1 to the samples;
Homogenize using a Retsch MM300 bead mill at 30 Hz for 5 min;
Centrifuge for 3 min at 13,000 x g;
Add 100 µl Buffer ST2;
Incubate for 10 min at 2-8 °C;
Centrifuge for 3 min at 13,000 x g;
Transfer 650 µl lysate to a NucleoSpin Stool Filter Plate sitting und on a NucleoVac 96 vacuum manifold;
Apply vacuum (-0,6 bar) until all liquid has passed the plate;
Collect cleared lysate in a 96well square well block;
Add 250 µl Buffer ST3;
Transfer 700 µl sample to a NucleoSpin Stool Binding Plate sitting und on a NucleoVac 96 vacuum manifold;
Apply vacuum (-0,6 bar) until all liquid has passed the plate;
Wash NucleoSpin Stool Binding Plate with 600 µl ST3, 600 µl ST4, 2 x 700 µl ST5; For each washing step, apply vacuum (-0,6 bar) until all liquid has passed the plate; Dry the membrane of the NucleoSpin Stool Binding Plate for 15 min at the maximum vacuum; and
Elute DNA with 150 µl Elution Buffer.

The yield was determined by absorption measurement at 260 nm, the results being shown in Table 1.

**Table 1**

| Sample No | Volume DNA/RNA shield | Yield/µg | Mean Yield/µg |
|---|---|---|---|
| 1 | 0 | 7,6 | 6,7 |
| 2 | | 6,8 | |
| 3 | | 5,7 | |
| 4 | | 6,6 | |
| 5 | 10 µl | 4,9 | 4,5 |
| 6 | | 4,3 | |
| 7 | | 5,0 | |
| 8 | | 3,7 | |
| 9 | 20 µl | 1,2 | 1,1 |
| 10 | | 1,1 | |
| 11 | | 1,2 | |
| 12 | | 1,1 | |
| 13 | 30 µl | 0,7 | 0,7 |
| 14 | | 0,7 | |
| 15 | | 0,5 | |
| 16 | | 0,8 | |
| 17 | 40 µl | 0,7 | 0,6 |
| 18 | | 0,6 | |
| 19 | | 0,4 | |
| 20 | | 0,7 | |

Conclusion: Even very low volumes of DNA/RNA Shield solution present during the lysis step reduce the yield of DNA drastically. The volume of 20-40 µl DNA/RNA Shield represent appox. 2-4% of the total lysis volume. This amount is already sufficient to reduce the average yield to 16-9%.

### Reference Example 2: Recovery of the DNA yield from DNA/RNA Shield^{™} stabilization solution containing stool samples via a pretreatment with NucleoZOL

Sample material: Samples 1-16 approx. 200 mg human stool sample in Bead Tubes Type A (MACHEREY-NAGEL); and Samples 17-24 approx. 400 mg human stool sample in Bead tubes Type A (as after the pretreatment only 50% of the sample was processed further, the initial sample input was doubled).
Sample treatment:

| | |
|---|---|
| Samples 1-8 | no addition of DNA/RNA Shield reagent (reference) |
| Samples 9-16 | addition of 100 µl DNA/RNA Shield reagent (negative control) |
| Samples 17-24 | addition of 500 µl DNA/RNA Shield reagent, mixing of stool |

sample and reagent followed by addition of 500 µl NucleoZOL
Pretreatment of samples 17-24: Vortex 2 min at maximum speed on a Vortex Genie 2; and transfer 500 µl into a fresh Bead Tube Type A
DNA-Extraction using the the NucleoSpin 96 DNA Stool kit (MACHEREY-NAGEL): Samples 1-16 add 950 µl Buffer ST1;
Samples 17-24 add 500 µl Buffer ST1;
Homogenize using a Retsch MM300 bead mill at 30 Hz for 5 min;
Centrifuge for 3 min at 13,000 x g;
Add 100 µl Buffer ST2;
Incubate for 10 min at 2-8 °C;
Centrifuge 3 min at 13,000 x g;
Transfer 650 µl lysate to a NucleoSpin Stool Filter Plate sitting und on a NucleoVac 96 vacuum manifold;
Apply vacuum (-0,6 bar) until all liquid has passed the plate;
Collect cleared lysate in a 96well square well block;
Add 250 µl Buffer ST3;
Transfer 700 µl sample to a NucleoSpin Stool Binding Plate sitting und on a NucleoVac 96 vacuum manifold (MACHEREY-NAGEL);
Apply vacuum (-0,6 bar) until all liquid has passed the plate;
Wash NucleoSpin Stool Binding Plate with 600 µl ST3, 600 µl ST4, 2 × 700 µl ST5; For each washing step, apply vacuum (-0,6 bar) until all liquid has passed the plate; Dry the membrane of the NucleoSpin Stool Binding Plate for 15 min at the maximum vacuum; and
Elute DNA with 150 µl Elution Buffer.

The yield was determined by absorption measurement at 260 nm, the results are shown in Table 2.

**Table 2:**

| Sample No | Volume DNA/RNA shield | Yield/µg | Mean Yield/µg |
|---|---|---|---|
| 1 | 0 (Reference) | 1,9 | 3,9 |
| 2 | | 3,0 | |
| 3 | | 5,2 | |
| 4 | | 4,2 | |
| 5 | | 4,6 | |
| 6 | | 3,5 | |
| 7 | | 4,4 | |
| 8 | | 4,1 | |
| 9 | 100 µl (Negative control) | 0,4 | 0,4 |
| 10 | | 0,5 | |
| 11 | | 0,4 | |
| 12 | | 0,4 | |
| 13 | | 0,4 | |
| 14 | | 0,3 | |
| 15 | | 0,6 | |
| 16 | | 0,4 | |
| 17 | 500 µl + 500 µl NucleoZOL | 1,4 | 4,0 |
| 18 | | 0,8 | |
| 19 | | 0,8 | |
| 20 | | 0,9 | |
| 21 | | 7,1 | |
| 22 | | 6,8 | |
| 23 | | 7,8 | |
| 24 | | 6,7 | |

Conclusion: With the pretreatment with NucleoZOL the average yield of DNA from stool samples can be restored, even in the presence of a large volume of DNA/RNA Shield reagent.

### Example 1: Recovery of the DNA yield from DNA/RNA Shield^{™} stabilization solution containing stool samples via a pretreatment with a 1:1 (v/v) mixture of eugenol and ethanol

Sample material: Four different human stool samples were aliquoted in triplicates into Bead Tubes Type A (MACHEREY-NAGEL).
Samples 1-12 approx. 200 mg human stool samples in Bead Tube Type A
Samples 13-24 approx. 400 mg human stool sample in Bead Tube Type A (as after the pretreatment only 43 % of the sample was processed further, the initial sample input was doubled).

Sample treatment:
Samples 1-12 no addition of DNA/RNA Shield reagent (reference)
Samples 13-24 addition of 500 µl DNA/RNA Shield reagent, mixing of stool sample and reagent followed by addition of 200 µl of the 1:1 (v/v) mixture of eugenol and ethanol.

### Pretreatment of samples 13-24: Vortex 2 min at maximum speed on a Vortex Genie 2; and transfer 300 µl into a fresh bead tube type A;

DNA-Extraction using the the NucleoSpin 96 DNA Stool kit:
Add 950 µl Buffer ST1;
Homogenize using a Retsch MM300 bead mill at 30 Hz for 5 min;
Centrifuge for 3 min at 13,000 x g;
Add 100 µl Buffer ST2;
Incubate for 10 min at 2-8 °C;
Centrifuge 3 min at 13,000 x g;
Transfer 650 µl lysate to a NucleoSpin Stool Filter Plate sitting und on a NucleoVac 96 vacuum manifold;
Apply vacuum (-0,6 bar) until all liquid has passed the plate;
Collect cleared lysate in a 96well square well block;
Add 250 µl Buffer ST3;
Transfer 700 µl sample to a NucleoSpin Stool Binding Plate sitting und on a NucleoVac 96 vacuum manifold;
Apply vacuum (-0,6 bar) until all liquid has passed the plate;
Wash NucleoSpin Stool Binding Plate with 600 µl ST3, 600 µl ST4, 2 × 700 µl ST5; For each washing step, apply vacuum (-0,6 bar) until all liquid has passed the plate; Dry the membrane of the NucleoSpin Stool Binding Plate for 15 min at the maximum vacuum; and
Elute DNA with 150 µl Elution Buffer.

The yield (in µg) was determined by absorption measurement at 260 nm, the results are shown in Table 3.

**Table 3:**

| | Fresh stool samples | | | | Stool samples dissolved in DNA/RNA Shield | | | |
|---|---|---|---|---|---|---|---|---|
| Replicate | Stool 1 | Stool 2 | Stool 3 | Stool 4 | Stool 1 | Stool 2 | Stool 3 | Stool 4 |
| 1 | 2,8 | 1,2 | 1,1 | 3,2 | 3,5 | 4,6 | 1,9 | 3,6 |
| 2 | 3,2 | 1,3 | 2,0 | 3,8 | 3,7 | 2,5 | 3,0 | 3,1 |
| 3 | 3,5 | 1,6 | 3,5 | 3,9 | 3,5 | 4,5 | 2,6 | 5,1 |
| mean value | 3,2 | 1,3 | 2,2 | 3,6 | 3,6 | 3,9 | 2,5 | 3,9 |

Conclusion: The average yield of DNA from samples that were dissolved in DNA/RNA Shield solution and pretreated with the 1:1 mixture of eugenol and ethanol is comparable or even slightly higher compared to the yield derived from fresh sample material. The mixture of eugenol and ethanol was able to neutralize the negative impact of the stabilization reagent on the extraction protocol and restore the yield of DNA, extracted from the stool samples.

Investigation of the integrity of the DNA via agarose gel electrophoresis: In order to investigate the integrity of the DNA isolated from the DNA/RNA shield solution based on the pretreatment with the mixture of Eugenol and ethanol in comparison to the integrity of DNA, isolated from reference samples, one replicate of each stool sample was analyzed on a 1% agarose TAE-gel. In Particular, 8 µl of one replicate from each fresh stool sample and stool samples dissolved in DNA/RNA Shield solution, were analyzed on a 1 % agarose TAE gel (Tris-Acetate-EDTA). The gel was run at 100 V constant voltage for approx. 45 min and stained with HDGreen Plus DNA/RNA stain (Intas Science Imaging).

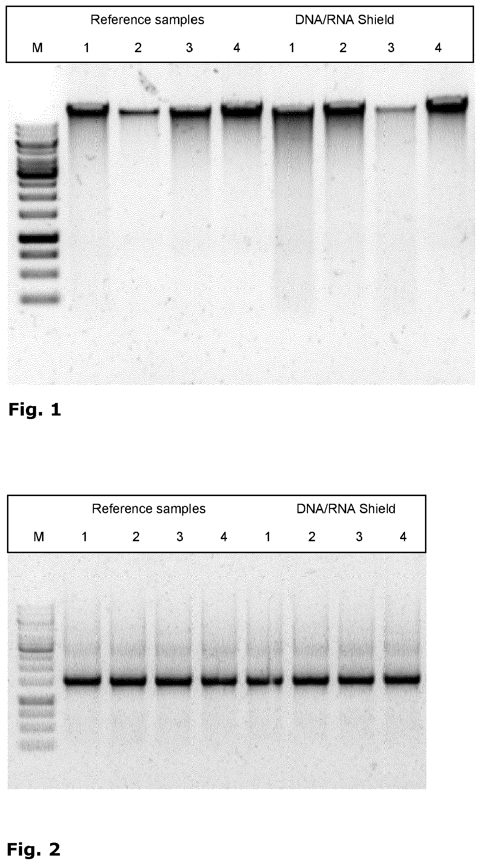

The results relative to M, the GeneRuler^{™}1kb Ladder (Thermo), are shown in Figure 1. The integrity of the DNA, extracted from reference samples and from stool samples dissolved in DNA/RNA Shield solution was equivalently high.

PCR-Analysis: In order to determine the quality of the isolated DNA one replicate of each stool sample was analyzed via end-point PCR. From each replicate, 2,5 µl of the eluate was subjected to the analysis of an 1,5 kb fragment of the bacterial 16S rRNA gene in a PCR reaction with 25 µl total volume using Biotaq^{™} DNA polymerase and reaction buffer.
Forward primer: AGAGTTTGATCCTGGCTCAG (SEQ ID NO:1)
Reverse primer: AAGGAGGTGATCCAGCCGCA (SEQ ID NO:2)

PCR-conditions: 5 min 95 °C; 35 x 40 s 95 °C / 30 s 55 °C / 60 s 72 °C; 5 min 72 °C. 5 µl of each PCR-reaction was analyzed on a 1 % agarose TAE gel. The gel was run at 80 V constant voltage for approx. 35 min and stained with HDGreen Plus DNA/RNA stain (Intas Science Imaging).

The results relative to M, the GeneRuler^{™}1kb Ladder, are shown in Fig.2. The 1,5 kb fragment of the 16SrRNA gene was amplified in all replicates, indicating that there were no interfering substances co-purified with the DNA and that the DNA was of high quality.

### Example 2: Extraction of DNA from a DNA/RNA Shield containing stool sample via a pretreatment with a 50 % (w/v) solution of thymol dissolved in ethanol

Thymol (2-isopropyl-5-methylphenol), is a second example for a phytochemical based on a phenol structure. As it is poorly soluble in water, it was used as a 50% (w/v) solution dissolved in ethanol.

Sample material: A human stool sample was aliquoted in quadruplicates into Bead Tubes Type A (MACHEREY-NAGEL).
Samples 1-4 approx. 200 mg human stool samples in bead tube type A
Samples 5-8 approx. 400 mg human stool sample in bead tube type A* *As after the pretreatment only 43 % of the sample was processed further, the initial sample input was doubled.

Sample treatment:
Samples 1-4 no addition of DNA/RNA Shield reagent (reference)
Samples 5-8 addition of 500 µl DNA/RNA Shield reagent, mixing of stool sample and reagent followed by addition of 200 µl of the solution of 50% (w/v) thymol dissolved in ethanol.

### Pretreatment of samples 5-8: Vortex 2 min at maximum speed on a Vortex Genie 2; and transfer 300 µl into a fresh bead tube type A

DNA-Extraction using the the NucleoSpin 96 DNA Stool kit:
Add 950 µl Buffer ST1;
Homogenize using a Retsch MM300 bead mill at 30 Hz for 5 min;
Centrifuge for 3 min at 13,000 x g;
Add 100 µl Buffer ST2;
Incubate for 10 min at 2-8 °C;
Centrifuge 3 min at 13,000 xg;
Transfer 650 µl lysate to a NucleoSpin Stool Filter Plate sitting und on a NucleoVac 96 vacuum manifold;
Apply vacuum (-0,6 bar) until all liquid has passed the plate;
Collect cleared lysate in a 96well square well block;
Add 250 µl Buffer ST3;
Transfer 700 µl sample to a NucleoSpin Stool Binding Plate sitting und on a NucleoVac 96 vacuum manifold;
Apply vacuum (-0,6 bar) until all liquid has passed the plate;
Wash NucleoSpin Stool Binding Plate with 600 µl ST3, 600 µl ST4, 2 x 700 µl ST5; For each washing step, apply vacuum (-0,6 Bar) until all liquid has passed the plate; Dry the membrane of the NucleoSpin Stool Binding Plate for 15 min at the maximum vacuum; and
Elute DNA with 150 µl Elution Buffer.

The yield (in µg) was determined by absorption measurement at 260 nm, the results are shown in Table 4.

**Table 4**

| Replicate | Reference samples | Stool sample dissolved in Zymo DNA/RNA Shield |
|---|---|---|
| 1 | 5,8 | 4,6 |
| 2 | 4 | 5,8 |
| 3 | 2,1 | 6,9 |
| 4 | 5,6 | 6,2 |
| Mean value | 4,4 | 5,9 |

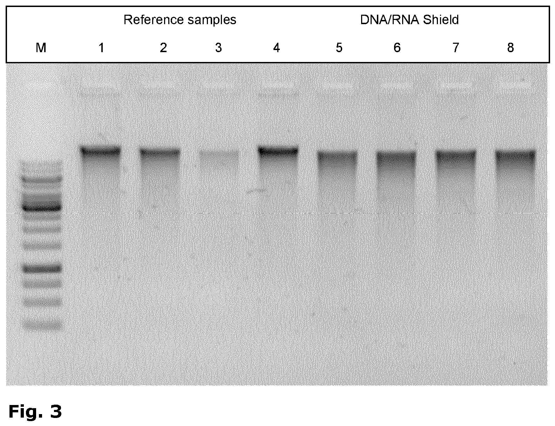

Analysis of the integrity of the DNA by agarose gel electrophoresis: In order to investigate the integrity of the DNA, isolated from fresh stool samples and stool samples dissolved in DNA/RNA Shield solution, 8 µl of each replicate were analyzed on a 1 % agarose TAE gel (Tris-Acetate-EDTA). The gel was run at 100 V constant voltage for approx. 45 min and stained with HDGreen Plus DNA/RNA stain. The results relative to M, the GeneRuler^{™}1kb Ladder, are shown in Figure 3.

Conclusion: The average yield of DNA from samples that were dissolved in DNA/RNA Shield solution and pretreated with the 50% (w/v) solution of Thymol dissolved in ethanol is comparable or even slightly higher compared to the yield derived from fresh sample material. Like for the Eugenol/Ethanol mixture, also the Thymol/Ethanol mixture was able to restore the yield of DNA extracted from stool dissolved in the DNA/RNA Shield solution. The integrity of the DNA, extracted from reference samples and from stool samples dissolved in DNA/RNA Shield solution was equivalently high.

## Claims

1. A method for the isolation of nucleic acids from chaotropic salt-containing biological samples, the method comprising adding to the crude chaotropic salt-containing biological sample, prior to separation of the nucleic acids from said solution, a masking solution comprising a C₃₋₁₀ alkyl or C₃₋₁₀ alkenyl phenol component in an organic solvent to mask chaotropic salt(s) present in the crude sample.

2. The method of claim 1, wherein the C₃₋₁₀ alkyl- or C₃₋₁₀ alkenylphenol component has the formula (I) 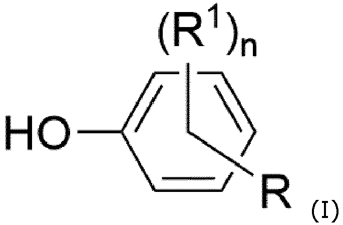 wherein
R is a straight- or branched-chain C₃₋₁₀ alkyl residue or C₃₋₁₀ alkenyl residue,
R¹ is independently selected from each other from a methyl, ethyl and methoxy group, and
n is an integer from 0 to 3,
preferably R is a straight- or branched-chain C₃₋₆ alkyl residue or C₃₋₆ alkenyl residue, R¹ is a methyl or methoxy group and n is 0 or 1, most preferably the C₃₋₁₀ alkyl- or C₃₋₁₀ alkenylphenol component is eugenol, thymol or carvacrol.

3. The method of claim 1 or 2, wherein
(i) the organic solvent is selected from alcohol, preferably is ethanol or propanol; and/or
(ii) the C₃₋₁₀ alkyl or C₃₋₁₀ alkenylphenol component is comprised in the organic solvent in at 10 to 90 % (w/v) or (v/v), preferably at 30 to 70 wt. or vol.% (w/v) or (v/v), most preferably at about 50 wt. or vol. % (w/v) or (v/v); and/or
(iii) the volume ratio of the crude sample to the masking solution is from 10:1 to 1:10, preferably from 4:1 to 1:4, most preferably at about 2.5:1; and/or
(iv) the weight ratio of chaotropic salt to the C₃₋₁₀ alkyl- or C₃₋₁₀ alkenylphenol component is from 3:1 to 1:3, preferably from 2:1 to 1:2, most preferably is about 1:1.

4. The method of any one of claims 1 to 3, wherein
(i) the crude chaotropic salt-containing biological sample is a stool sample, cell lysate, environmental- or blood sample; and/or
(ii) the nucleic acids isolated are selected from DNA and RNA.

5. The method of any one of claims 1 to 4, wherein the method comprises
(a) providing the crude chaotropic salt-containing biological sample;
(b) adding an appropriate amount of a masking solution as defined in any one of claims 1 to 3;
(c) optionally mechanically disrupting and clearing the masking solution containing sample of step (b);
(d) adding a binding solution to the solution obtained in step (b) or (c), bringing the resulting solution into contact with a nucleic acid-binding solid phase and separating the solid phase with the nucleic acids bound thereto from the remainder of the solution;
(e) washing the separated solid phase with the nucleic acids bound thereto at least once with same or different washing solutions; and
(f) desorbing the nucleic acids from the washed solid phase by adding an elution buffer.

6. The method of claim 4 or 5, which is for the separation of nucleic acids from stool samples and comprises a disrupting and clearing step (c), preferably the clearing step (c) comprises adding a dilution solution, mixing/incubating the resulting solution, centrifuging the resulting solution and subjecting an aliquot of the resulting supernatant to a filter device, most preferably the dilution solution comprises a non-chaotropic salt.

7. The method of claim 4 or 5, which is for the separation of nucleic acids from cell lysates stabilized with chaotropic salts.

8. The method of any one of claims 5 to 7, wherein
(i) the binding solution is an aqueous high salt solution, preferably containing guanidiniumthiocyanate and a citrate buffer; or
(ii) the binding solution is aqueous high salt solution, optionally containing an alcohol and/or ionic or non-ionic detergent; and/or
(iii) the nucleic acid-binding solid phase is selected from a silica-matrix/membrane or magnetic particles.

9. The method of any one of claims 5 to 8, wherein the washing solutions of step (e) are selected from washing solutions containing an alcohol, a chaotropic salt or mixtures thereof.

10. The method of any one of claims 5 to 9, wherein the elution buffer of step (f) is a slightly alkaline low-salt buffer, preferably the volume of the elution buffer is about 10 to about 20 times the volume of the nucleic acid-binding solid phase employed in step (d).

11. A kit for isolating nucleic acids from a biological sample, comprising a masking solution comprising a C₃₋₁₀ alkyl or C₃₋₁₀ alkenyl phenol component in an organic solvent to mask chaotropic salt(s) present in the crude biological sample.

12. The kit of claim 11, which is suitable for performing a method according to any one of claims 1-10, preferably
(i) is for the separation of nucleic acids from stool samples and optionally further contains one or more of a dilution solution, a stool filter, a binding solution, a nucleic acid-binding solid phase, washing solutions and an elution buffer as defined in claims 6 and 8 to 11; or
(ii) is for the separation of nucleic acids from cell lysates and optionally further contains one or more of a chaotropic salt-containing lysis solution, chaotropic salt-containing stabilizing solution, a binding solution, a nucleic acid-binding solid phase, washing solutions and an elution buffer as defined in claims 7 to 11.

13. Use of a masking solution comprising a C₃₋₁₀ alkyl or C₃₋₁₀ alkenyl phenol component in an organic solvent to mask chaotropic salt(s) present in crude biological samples in a nucleic acid isolation method.

14. The use of claim 13, wherein the masking solution, biological sample and/or isolation method are as defined in claims 1 to 5.

## Amended claims

### Amended claims in accordance with Rule 137(2) EPC.

1. A method for the isolation of nucleic acids from chaotropic salt-containing stool samples, the method comprising adding to the crude chaotropic salt-containing stool sample, prior to separation of the nucleic acids from said solution, a masking solution comprising a phenol component having the formula (I) 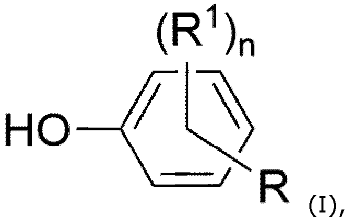 wherein R is a straight- or branched-chain C₃₋₆ alkyl residue or C₃₋₆ alkenyl residue, R¹ is a methyl or methoxy group and n is 0 or 1, in an alcoholic solvent to mask chaotropic salt(s) present in the crude sample.

2. The method of claim 1, wherein the phenol component is eugenol, thymol or carvacrol.

3. The method of claim 1 or 2, wherein
(i) the alcoholic solvent is is ethanol or propanol; and/or
(ii) the phenol component is comprised in the alcoholic solvent in at 10 to 90 % (w/v) or (v/v), preferably at 30 to 70 wt. or vol.% (w/v) or (v/v), most preferably at about 50 wt. or vol. % (w/v) or (v/v); and/or
(iii) the volume ratio of the crude sample to the masking solution is from 10:1 to 1:10, preferably from 4:1 to 1:4, most preferably at about 2.5:1; and/or
(iv) the weight ratio of chaotropic salt to the phenol component is from 3:1 to 1:3, preferably from 2:1 to 1:2, most preferably is about 1:1.

4. The method of any one of claims 1 to 3, wherein the nucleic acids isolated are selected from DNA and RNA.

5. The method of any one of claims 1 to 4, wherein the method comprises
(a) providing the crude chaotropic salt-containing stool sample;
(b) adding an appropriate amount of a masking solution as defined in any one of claims 1 to 3;
(c) optionally mechanically disrupting and clearing the masking solution containing stool sample of step (b);
(d) adding a binding solution to the solution obtained in step (b) or (c), bringing the resulting solution into contact with a nucleic acid-binding solid phase and separating the solid phase with the nucleic acids bound thereto from the remainder of the solution;
(e) washing the separated solid phase with the nucleic acids bound thereto at least once with same or different washing solutions; and
(f) desorbing the nucleic acids from the washed solid phase by adding an elution buffer.

6. The method of claim 4 or 5, which comprises a disrupting and clearing step (c).

7. The method of claim 6, wherein the clearing step (c) comprises adding a dilution solution, mixing/incubating the resulting solution, centrifuging the resulting solution and subjecting an aliquot of the resulting supernatant to a filter device, preferably the dilution solution comprises a non-chaotropic salt.

8. The method of any one of claims 5 to 7, wherein
(i) the binding solution is an aqueous high salt solution, preferably containing guanidiniumthiocyanate and a citrate buffer; or
(ii) the binding solution is aqueous high salt solution, optionally containing an alcohol and/or ionic or non-ionic detergent; and/or
(iii) the nucleic acid-binding solid phase is selected from a silica-matrix/membrane or magnetic particles.

9. The method of any one of claims 5 to 8, wherein the washing solutions of step (e) are selected from washing solutions containing an alcohol, a chaotropic salt or mixtures thereof.

10. The method of any one of claims 5 to 9, wherein the elution buffer of step (f) is a slightly alkaline low-salt buffer, preferably the volume of the elution buffer is about 10 to about 20 times the volume of the nucleic acid-binding solid phase employed in step (d).

11. A kit for isolating nucleic acids from a stool sample, comprising a masking solution comprising a phenol component having the formula (I) 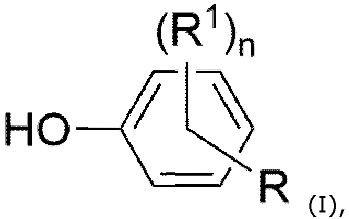 wherein R is a straight- or branched-chain C₃₋₆ alkyl residue or C₃₋₆ alkenyl residue, R¹ is a methyl or methoxy group and n is 0 or 1, in an alcoholic solvent to mask chaotropic salt(s) present in the crude stool sample.

12. The kit of claim 11, which is suitable for performing a method according to any one of claims 2-10 and optionally further contains one or more of a dilution solution, a stool filter, a binding solution, a nucleic acid-binding solid phase, washing solutions and an elution buffer as defined in claims 6 to 10.

13. Use of a masking solution comprising a phenol component having the formula (I) 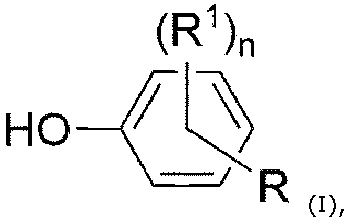 wherein R is a straight- or branched-chain C₃₋₆ alkyl residue or C₃₋₆ alkenyl residue, R¹ is a methyl or methoxy group and n is 0 or 1, in an alcoholic solvent to mask chaotropic salt(s) present in crude stool samples in a nucleic acid isolation method.

14. The use of claim 13, wherein the masking solution and/or isolation method are as defined in claims 1 to 5.
